(19) Europäisches Patentamt
European Patent Office
Office européen des brevets

(11) Publication number: **0 457 960 A2**

# (12) EUROPEAN PATENT APPLICATION

(21) Application number: 90121176.3

(22) Date of filing: 06.11.90

(51) Int. Cl.5: **A61K 47/02, A61K 47/12, A61M 1/16, A61M 1/28, A61L 2/00**

(30) Priority: 17.05.90 US 525114

(43) Date of publication of application: 27.11.91 Bulletin 91/48

(84) Designated Contracting States: AT BE CH DE DK ES FR GB IT LI LU NL SE

(71) Applicant: **Minntech Corporation**
**14905 28th Avenue North**
**Minneapolis Minnesota 55441(US)**

(72) Inventor: **Consentino, Louis C.**
**2435 Holly Lane**
**Plymouth, MN 55447(US)**
Inventor: **Baker, Daniel A.**
**12900 St. David's Road**
**Minnetonka, MN 55343(US)**
Inventor: **Jansen, Walter B.**
**4015 Evergreen Lane**
**Plymouth, MN 55441(US)**

(74) Representative: **Eisenführ, Speiser & Strasse**
**Martinistrasse 24**
**W-2800 Bremen 1(DE)**

(54) **High acid concentration dialysates.**

(57) A dialysis fluid made by combining an alkalizer/buffer with an acid concentrate to form an aqueous material having, in a 45X machine fluid at least 3 millimoles/liter acid, and in a 36.83X machine fluid at least 5 millimoles/liter of an acid. The dialysis fluid is useful in common dialysis equipment and is stable during manufacture, storage, and use.

## Field of the Invention

The invention relates to the composition of an aqueous dialysate fluid used in the machine hemodialysis of human blood, most commonly, in the treatment of patients having end-stage renal failure. More particularly, the invention resides in a dialysate fluid system that can be used in available hemodialysis units at typical blending ratios or reagent proportions. The dialysate system can be used without the drawbacks caused by the formation, in the dialysate system, of precipitation products such as carbonate salts or other precipitate contamination.

## Background of the Invention

Individuals requiring renal system support, or who have end-stage renal disease or renal failure, have kidneys temporarily or permanently incapable of removing products of metabolism and other substances from the blood for excretion in urine. Products of metabolism or metabolites typically include such compounds as urea, creatinine, natural biochemical metabolites, drug metabolites, excess electrolytes, etc. Individuals with end-stage renal disease have the option of either undergoing the replacement of a diseased kidney through the transplant of a healthy kidney, or undergoing periodic hemodialysis (multiple weekly treatments) to reduce the concentration of harmful materials in the blood stream. Other individuals need hemodialysis for brief periods of renal support.

Hemodialysis is a process in which solute molecules, which constitute undesirable waste products in human blood, can be transported (i.e., removed from the blood stream) across a membrane into a dialysis fluid. The driving forces of such transport are (1) the difference in hydrostatic pressure across the membrane and (2) the difference in chemical potential of each individual solute molecule across the membrane. Dialysis requires that membranes separating blood from dialysis fluid permit diffusional transfer of at least some of the molecular species present in blood into the fluid while effectively preventing any return contamination of blood or commingling of the blood and the dialysis fluid. Dialysis is a passive separation process with low operating costs using no external thermal or chemical energy sources. The basic hemodialysis separation obtained is between large cells and molecules, such as red blood cells (RBC), white blood cells (WBC) and proteins, and small molecules such as urea, electrolytes, and other small molecule metabolites. To obtain reasonable separation rates, machines are designed with large areas of membranes and are used at relatively small flow rates. Modern hemodialysis machines utilize small diameter hollow-fiber dialysis membranes bathed in dialysate fluids. Such membranes are in the form of a cylindrical cartridge having a large number of hollow-fiber membranes in fluid connection with a blood inlet and outlet. The blood flows through the hollow interior membrane space. The hollow-fiber membrane exterior is bathed in dialysate fluid. Hollow-fiber membranes are typically manufactured from polymers such as cellulose acetate, cellulose ester, polysulfone, polyacrylonitrile, polymethylmethacrylate, polyamide, polyimidazole, hollow-fiber glass, etc.

Dialysis fluids are prepared with a pH and composition compatible with blood. The pH of the blood should not significantly be changed during the procedure and no precipitate should be formed in the dialysis fluid. The production of even small quantities of precipitate can adversely affect the operation of the machine.

The dialysis fluids currently commercially available are formed by diluting an acid concentrate and an alkalizer/buffer in a water stream. Typical fluids are based on the combination of sodium bicarbonate, acetic acid, and other components. Conventional dialysate fluids, upon dilution, use acid at a concentration equal to or less than about 4 millimoles of acid per liter (36.83X machine) and equal to or less than about 2 millimoles (45X machine) of acid per liter. These dialysate fluids are not free of drawbacks including pH instability.

A major drawback regarding functional dialysate fluids used in the hemodialysis machines relates to the tendency of the fluid to form precipitates during operation. Such precipitates typically take the form of calcium salts resulting from the presence of bicarbonate ion ($HCO_3^-$), o r carbonate ion ($CO_3^{-2}$) in the dialysate fluid at pHs common during operation.

Accordingly, a substantial need exists for development of improved non-precipitate forming dialysis fluids for use in machines using hollow-fiber technology.

## Brief Discussion of the Invention

We have found that the tendency of dialysate fluids to generate precipitates can be substantially reduced by increasing the concentration of acid in the dialysate fluid and in the dialysate concentrate used to prepare the fluid, and by operating the hemodialysis process with the fluid, at a higher acid concentration than conventional dialysates. The dialysate fluids of the invention are typically prepared in a three-stream dilution by combining an alkalizer/buffer concentrate stream and an acid concentrate stream with a stream comprising an aqueous, non-pyrogenic, stable, bacteria-free diluent. Such a three-stream system, when operated at appropriate ratios of alkalizer/buffer to diluent and acid, produces the useful dialysate composition of the invention.

## Brief Discussion of the Drawings

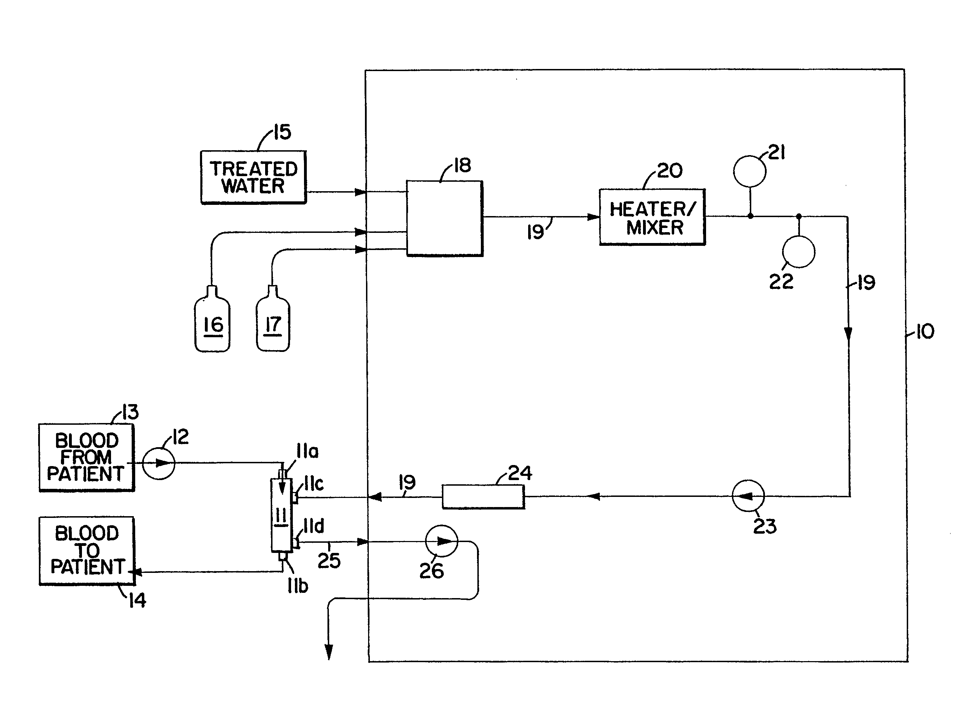

Figure 1 is a block diagram of typical hemodialysis equipment containing machine elements for the preparation of the dialysate fluid and elements to direct fluid to the membrane within the artificial kidney to remove contaminants from patient's blood.

## Detailed Description of the Invention

In the preparation of a dialysate fluid used in common hemodialysis equipment, an alkalizer/buffer concentrate and an acid concentrate are added to an aqueous diluent stream at appropriate mixing ratios to produce the useful dialysate fluid which is directed to the dialysis membrane. The acid concentrate has a pH less than about 3.0, while the alkalizer/buffer has a pH greater than about 8.0. When diluted at a usual machine ratio, the pH of the mixed dialysate is below the pH at which $CaCO_3$ will precipitate. Over time, the pH will increase as the bicarbonate buffering reactions take place, but precipitation will not take place in the machine. The source of bicarbonate ion typically comprises alkali metal bicarbonate salts. Such materials in aqueous solution typically yield bicarbonate ($HCO_3^-$) ion and, depending upon pH, some free carbonate ($CO_3^{-2}$) ion. The relative proportions of carbonate and bicarbonate ions typically depend on the pH of the alkalizer/buffer solution and the amount of bicarbonate salt contained in the alkalizer/buffer solution. The alkalizer/buffer solution must contain, at a minimum, a source of bicarbonate ion. The alkalizer/buffer material can contain other ionized and non-ionized solutes that are compatible in aqueous solution with the dialysis product and the source of carbonate and bicarbonate ion. However, preferably, the alkalizer/buffer concentrate consists essentially of a source of bicarbonate as a source of alkalinity. The alkalizer/buffer solution contains little or no other materials that significantly contribute to the alkaline pH of the alkalizer/buffer material. The amount of bicarbonate in the concentrate depends primarily on the blending ratio of concentrate to diluent stream used in the hemodialysis dialysate fluid. The blending ratio is fixed in each machine type and the concentrations are formulated for the machine blending ratio. The acid concentrate material used in preparing the dialysate fluid is typically prepared in aqueous solution in combination with other solutes compatible with the acid. The amount of acid used in the acid concentrate also depends on the hemodialysis machine used and the blending ratio characteristic of the machine. The acid concentrate material of the invention can contain an acid in sufficient amount to result in an addition of at least 3 millimolar acid for the dialysate fluid in a 45X machine or at least 5 millimolar acid for the dialysate fluid used in a 36.83X machine, or higher, depending on the operating concentration selected. Preferably, the acid, in the concentrations used in the dialysate fluid, produces little or no adverse physiological impact on the blood present in the hemodialysis machine or on the patient undergoing dialysis. More preferably, the acid has an acid cation which is identical to or sufficiently related to cations present in normal human metabolism. Such acids include but are not limited to hydrochloric acid (HCl), acetic acid ($HC_2H_3O_2$, HOAc), citric acid ($H_3C_6H_5O_7$), lactic acid ($HC_3H_5O_3$), pyruvic acid ($HC_3H_3O_3$), formic acid ($HCHO_2$), fumaric acid ($H_2C_4H_2O_4$), succinic acid ($H_2C_4H_4O_4$), and others.

As set forth above, the concentration of acid in the concentrates diluted to form the dialysate fluids of the invention are selected at a concentration sufficiently high to result in adding at least 5 millimolar acid (36.83X machine) or at least 3 millimolar acid (45X machine), into the final dialysis fluid concentration which can effectively prevent the production of harmful precipitates. For example, dialysate fluids of the present invention may be prepared from an acid concentrate material comprising at least 135 millimolar acid for the 45X machine and at least 180 millimolar acid for the 36.83X machine.

The dialysate fluid of the invention can also contain a variety of other physiological solutes and other solutes useful in treating the dialysis patient. Solutes common in humans include sodium, potassium, chloride, calcium, magnesium, phosphate, dextrose, sulfate, iron, copper, and others. The amount of such materials in the dialysate fluid is adjusted for the requirements of the individual dialysis patient. For example, the amount of dextrose used must be tailored for the diabetic patient. The amounts of Ca++, Na+, and K+ must be tailored for the cardiac patient. The dialysate fluids can contain, in each liter, from about 90 to 155 milliequivalents of sodium ion, 0 to 6, preferably 0.1 to 5 milliequivalents of potassium ion, 0 to 6, preferably 0.1 to 5 milliequivalents of calcium ion, 0 to 6, preferably 0.1 to 5 milliequivalents of magnesium ion, 70 to 300 milliequivalents of chloride ion, 20 to 40 milliequivalents of bicarbonate ion, etc. The fluid can also contain 0 to 500, preferably 50 to 400 milligrams of dextrose per 100 ml of dialysate fluid. Other solutes present in the dialysate solution can be tailored to the specific patient.

Other materials can be placed in the dialysate solution to treat the blood of the dialysis patient. For example, the dialysate solution can contain hormones, antibiotics, anticoagulants, and other nutrients. Useful hormones include materials such as insulin, steroids, prostaglandins, etc.; antibiotics include penicillin, cephlo-sporins, etc.; anticoagulants such as heparin, coumarin anticoagulants, indan-1,3-dione coagulants; and nutrients such as amino acids, dextrose, vitamins, etc.

Machines used in hemodialysis contain sensors, pumps, and control units that control the flow rate, flow direction, fluid pressure, fluid temperature, dilution ratios of the buffer alkalizer concentrate and the acid concentrate into the diluent water stream.

## Detailed Description of the Drawing

Figure 1 shows a block diagram of a typical dialysis machine. The artificial kidney or membrane cartridge 11 is shown having an inlet 11a and an outlet 11b to permit the flow of blood through the cartridge. Blood comes from the patient 13 through a blood pump 12 through the cartridge 11 and returns to the patient 14. The dialysate fluid is blended in an proportioning pump 18 by combining treated diluent water 15, acid concentrate 16, and bicarbonate (alkalizer/buffer) concentrate 17. These materials are blended at a machine set dilution rate (36.8X or 45X) to produce a final dialysate stream 19. The dialysate stream 19 passes through a heater/mixer 20 which ensures uniformity and physiological temperature. The proper operation of the dialysis machine is monitored by a conductivity monitor 21, thermometer 22, and pressure regulator 23 and flow meter 24. The dialysate fluid stream 19 passes into the cartridge 11 through inlet 11c, wherein the fluid contacts the exterior of the hollow-fiber dialysis membrane containing the blood in the internal membrane passage. The dialysate receives material by diffusion from the blood across the membrane. The contaminated dialysate 25 leaves the cartridge at outlet 11d due to negative pressure from pump 26 and is directed to a drain.

In the operation of the hemodialysis unit, the alkalizer/buffer material is blended within a three-stream dilution unit by first blending the alkalizer/buffer and water to form a diluted alkalizer buffer. The acid concentrate is then blended with the diluted alkalizer buffer and mixed until uniform. The ratio of components are about 1.6 to 3.3 parts of alkalizer/buffer per part of acid concentrate and about 30 to 50 parts of purified water (pyrogen-free, bacteria-free, U.S.P. or equivalent).

In a 36.83X machine, the dialysate is formed by first blending the acid concentrate and purified water to form a diluted acid. The alkalizer/buffer is then blended with the diluted acid to form the final dialysate fluid. The ratio of components are about 1 part of acid concentrate per each 1.7 to 2.5 parts alkalizer buffer and about 1 part of acid per each 30 to 40, preferably 34 parts (volume) of purified water. The dialysate fluid is blended by combining the three streams in a blending unit and is directly introduced into the housing or cartridge holding the hollow-fiber membranes (artificial kidney). The fluid obtains the impurities from the blood held in the interior of the hollow fiber by diffusion through the membrane's surface to the exterior dialysate fluid. The contaminated fluid is directed out of the housing to a drain.

The blending equipment of the hemodialysis unit is operated at a rate such that about 250 to 1000 ml of dialysate fluid are delivered to the exterior surface hollow fiber membrane (artificial kidney) unit per minute having the constituents proportioned as described above. The preferred rate of dilution of the concentrates and purified water results in a stream of about 400 to 800 ml of dialysate fluid per minute.

Proper proportioning of the dialysate constituents during dilution and subsequent hemodialysis can readily be monitored by a conductivity sensor because the relative conductivities of the aqueous diluent (low conductivity) and the concentrates (high conductivity ) are significantly different from the conductivity of the diluted fluid. Should the dilution system fail and deliver diluent or concentrate to the dialysis unit, conductivity sensors can monitor and terminate flow at that time. The conductivity of the fluid should preferably be maintained at about 13.0 to 14.5 milliSiemens.

An illustrative dialysate fluid (HCl-based) prepared from the acid concentrate that is at least 3 or 4.5 millimolar in acid (45X machine) or 5 or 6.5 millimolar in acid (36.83X machine) can contain the following constituents after blending:

| Ion | Concentration |
|---|---|
| Na+ | 90-155 mEq/L |
| $HCO_3-$ | 25-45 mEq/L |
| Cl- (salt) Cl- | 110-119 mEq/L |
| Ca++ | 0-6 mEq/L |
| | (preferably, 0.1-5 mEq/L) |
| Mg++ | 0-6 mEq/L |
| | (preferably, 0.1-2.5 mEq/L) |

Alternatively, a mixed acid/acetic acid dialysate fluid can be prepared from the acid concentrate, that is at least 5, or at least 6 millimolar total acid, of the invention having the following composition after blending:

| Ion | Concentration |
|---|---|
| Na+ | 90-155 mEq/L |
| $HCO_3-$ | 25-45 mEq/L |
| $C_2H_3O_2-$ | 0.1-10 mEq/L |
| Cl- (salt) | 100-117 mEq/L |
| Ca++ | 0-6 mEq/L |
| | (preferably, 0.1-5 mEq/L) |
| Mg++ | 0-6 mEq/L |
| | (preferably, 0.1-2.5 mEq/L) |

These illustrative dialysate fluids are prepared from an acid concentrate and an alkalizer/buffer. The preferred alkalizer/buffer material used in the invention contains about 50 to 95 grams of sodium bicarbonate per liter of material or can contain about 10 to 30 grams of sodium chloride and 40 to 90 grams sodium bicarbonate. Preferably, the alkalizer/buffer material contains about 80 to 85 grams of sodium bicarbonate per liter.

Typical aqueous acid concentrates suitable for combination with the alkalizer/buffer material set forth above are prepared as illustrated hereafter:

**<u>Preparation 1</u>** (36.83X Dilution)

| Ingredient | Concentration |
|---|---|
| NaCl | 172.2 gm/L |
| HCl (12.1 <u>N</u>) | 18.2 gm/L |
| KCl | 5.49 gm/L |
| dextrose | 73.66 gm/L |
| water | q.s. |
| (pH 0 to 2.0) | |

Expressed in different terms:

| Cl- | 3204.25 mEq/L |
|---|---|
| Na+ | 2946.4 mEq/L |
| K+ | 73.7 mEq/L |
| dextrose | 736.6 mgm/dL |

**Preparation 2** (36.83X Dilution)

| Ingredient | Concentration | |
|---|---|---|
| NaCl | 172.2 | gm/l |
| HCl (12.1 $\underline{N}$) | 20.0 | gm/L |
| KCl | 5.49 | gm/L |
| MgCl.6$H_2$O | 3.74 | gm/L |
| dextrose | 73.66 | gm/L |
| CaCl$_2$.2$H_2$O | 9.48 | gm/L |
| water | q.s. | |
| (pH 0 to 2.0) | | |

Expressed in different terms:

| | | |
|---|---|---|
| Cl- | 3388.4 | mEq/L |
| Mg++ | 36.8 | mEq/L |
| Na+ | 2946.4 | mEq/L |
| Ca++ | 128.9 | mEq/L |
| K+ | 73.7 | mEq/L |
| dextrose | 736.6 | mgm/dL |

**Preparation 3** (36.83X Dilution)

| Ingredient | Concentration | |
|---|---|---|
| NaCl | 172.2 | gm/L |
| HCl (12.1 $\underline{N}$) | 23.6 | gm/L |
| KCl | 5.49 | gm/L |
| CaCl$_2$.2$H_2$O | 9.48 | gm/L |
| dextrose | 73.66 | gm/L |
| MgCl$_2$.6$H_2$O | 3.74 | gm/L |
| water | q.s. | |
| (pH 0 to 2.0) | | |

Expressed in different terms:

| | | |
|---|---|---|
| Cl- | 3425.2 | mEq/L |
| Na+ | 2946.4 | mEq/L |
| Ca++ | 128.9 | mEq/L |
| K+ | 73.7 | mEq/L |
| Mg++ | 26.8 | mEq/L |
| dextrose | 736.6 | mgm/dL |

**Preparation 4** (45X Dilution)

| Ingredient | Concentration | |
|---|---|---|
| NaCl | 263.0 | gm/L |
| HCl (12.1 $\underline{N}$) | 13.4 | gm/L |
| KCl | 6.7 | gm/L |
| $MgCl_2.6H_2O$ | 3.4 | gm/L |
| dextrose | 90.0 | gm/L |
| $CaCl_2.2H_2O$ | 9.9 | gm/L |
| water | q.s. | |
| (pH 0 to 3.0) | | |

Expressed in different terms:

| | | |
|---|---|---|
| Cl- | 4893.8 | mEq/L |
| Na+ | 4500.0 | mEq/L |
| K+ | 90.0 | mEq/L |
| Mg++ | 33.8 | mEq/L |
| Ca++ | 135.0 | mEq/L |
| dextrose | 900.0 | mgm/dL |

**Preparation 5** (45X Dilution)

| Ingredient | Concentration | |
|---|---|---|
| NaCl | 263.0 | gm/L |
| HCl (12.1 $\underline{N}$) | 17.7 | gm/L |
| $CaCl_2.2H_2O$ | 9.9 | gm/L |
| $MgCl_2.6H_2O$ | 3.4 | gm/L |
| KCl | 6.7 | gm/L |
| dextrose | 90.0 | gm/L |
| water | q.s. | |
| (pH 0 to 3.0) | | |

Expressed in different terms:

| | | |
|---|---|---|
| Cl- | 4938.8 | mEq/L |
| Na+ | 4500.0 | mEq/L |
| K+ | 90.0 | mEq/L |
| dextrose | 900.0 | mgm/dL |
| Ca++ | 135.0 | mEq/L |
| Mg++ | 33.8 | mEq/L |

**Preparation 6** (45X Dilution)

| Ingredient | Concentration | |
|---|---|---|
| NaCl | 263.0 | gm/L |
| HCl (12.1 $\underline{N}$) | 13.4 | gm/L |
| Glacial acetic acid | 5.4 | gm/L |
| $CaCl_2.2H_2O$ | 9.9 | gm/L |
| $MgCl_2.6H_2O$ | 3.4 | gm/L |
| KCl | 6.7 | gm/L |
| dextrose | 90.0 | gm/L |
| water | q.s. | |

Expressed in different terms:

| | | |
|---|---|---|
| Cl- | 4893.8 | mEq/L |
| Ca++ | 135.0 | mEq/L |
| Mg++ | 33.8 | mEq/L |
| Na+ | 4500.0 | mEq/L |
| K+ | 90.0 | mEq/L |
| dextrose | 900.0 | mgm/dL |

We have found that these preparations provide the efficient, non-contaminating, non-precipitating operation of a hemodialysis machine providing effective removal of solutes from blood. We have found that the solutions are typically stable and are not likely to promote the growth of microorganisms over typical manufacturer transportation storage and use periods. We have also tested the solutions of the invention and have found that the solutions are non-precipitating and can be used in common hemodialysis machines without a substantial reduction in the operating efficiency of semipermeable hollow-fiber membrane units.

The above discussion, preparations and figures describe the invention with respect to the current and preferred embodiments developed to date. However, those skilled in the art will understand that a variety of modifications and variations of the invention may be derived without departing from the essence or spirit and scope of the invention. Therefore, the invention resides in the claims hereinafter appended.

**Claims**

1. A dialysate fluid for use in an approximately 36.83X hemodialysis machine, resistant to the formation of a precipitate during dialysis, which comprises an aqueous physiologically compatible dilution product formed by combining (i) an alkalizer/buffer containing a source of alkalinity comprising aqueous bicarbonate; and (ii) an acid concentrate comprising an acid in sufficient amount to result in an addition of at least 5 millimolar acid in the dialysate fluid.

2. The fluid of claim 1 wherein the dilution product is formed by combining an aqueous diluent, an acid concentrate, and the alkalizer/buffer which contains a source of alkalinity consisting essentially of aqueous bicarbonate.

3. The fluid of claim 2 wherein the fluid is blended by combining the acid concentrate and purified water to form a diluted acid and then blending the diluted acid with alkalizer buffer at a fluid ratio of about 1.7 to 2.5 parts of alkalizer buffer and 30 to 40 parts of purified water and per part of acid concentrate.

4. A dialysis fluid for use in a approximately 36.83X hemodialysis machine, resistant to the formation of a precipitate during dialysis, which comprises:
   (a) about 90-155 mEq/L sodium ion; and
   (b) about 25-45 mEq/L bicarbonate ion; which is formed by combining:

(i) an alkalizer/buffer containing a source of alkalinity consisting essentially of aqueous sodium bicarbonate; and

(ii) an acid concentrate comprising an acid selected from the group consisting of hydrochloric acid, acetic acid, citric acid, lactic acid, pyruvic acid, formic acid, fumaric acid, succinic acids, and mixtures thereof in sufficient amount to result in an addition of at least 5 millimolar acid in the dialysate fluid.

5. The fluid of claim 4 wherein the dilution product is formed by combining an aqueous diluent with the acid concentrate and then with the alkalizer/buffer.

6. The fluid of claim 5 wherein the dilution is formed at a fluid ratio of about 1.7 to 2.5 parts of the alkalizer/buffer and about 30-40 parts of aqueous diluent are combined with each part of acid concentrate.

7. The fluid of claim 4 wherein the acid concentrate further comprises magnesium ion and calcium ion in an amount sufficient to result in the addition of about 0.1-6 mEq/L, independently, of each ion in the dialysate fluid.

8. A dialysis fluid for use in an approximately 36.83X machine, resistant to the formation of a precipitate during dialysis, which comprises an aqueous dilution product comprising about 90-155 mEq/L sodium ion, 25-45 mEq/L bicarbonate ion, and a physiologically acceptable concentration of a solute selected from the group consisting of sodium, potassium, calcium, magnesium ion and dextrose, which is formed by combining:

(i) an alkalizer/buffer containing a source of alkalinity consisting essentially of about 50-90 grams of sodium bicarbonate per liter of alkalizer/buffer; and

(ii) an acid concentrate comprising hydrochloric acid in sufficient amount to result in an addition of at least 5 millimolar hydrochloric acid in the dialysate fluid.

9. The fluid of claim 8 wherein the dilution product is formed by combining an aqueous diluent with the acid concentrate and the alkalizer/buffer.

10. The fluid of claim 9 wherein the dilution is formed at a fluid ratio of about 1.7 to 2.5 parts of the alkalizer buffer and about 30-40 parts of the aqueous diluent are combined with each part of the acid concentrate.

11. A method of hemodialysis of human blood which comprises:

(a) introducing human blood into the interior of a hollow fiber hemodialysis membrane in an approximately 36.83X hemodialysis machine; and

(b) contacting the exterior of the membrane with a dialysis fluid resistant to the formation of a precipitate during dialysis which comprises an aqueous dilution product formed by combining:

(i) an alkalizer/buffer containing a source of alkalinity comprising aqueous bicarbonate; and

(ii) an acid concentrate comprising an acid in sufficient amount to result in an addition of at least 5 millimolar acid in the dialysate fluid; and

(c) withdrawing the dialysate from the membrane and returning the blood to a patient.

12. The method of claim 11 wherein the dilution product is formed by combining an aqueous diluent with an acid concentrate and the alkalizer/buffer which contains a source of alkalinity consisting essentially of aqueous bicarbonate.

13. The method of claim 11 wherein the dialysate fluid is contacted with a membrane at a rate of about 200 to 1000 milliliters of fluid per minute.

14. The method of claim 11 wherein the acid concentrate further comprises magnesium ion and calcium ion in an amount sufficient to result in about 0.1 to 6 mEq/L, independently, of each ion in the dialysate fluid.

15. A dialysis fluid for use in an approximately 45X hemodialysis machine, resistant to the formation of a precipitate during dialysis, which comprises an aqueous dilution product formed by combining:

(i) an alkalizer/buffer containing a source of alkalinity consisting essentially of aqueous bicarbonate; and

(ii) an acid concentrate comprising an acid in sufficient amount to result in an addition of at least 3 millimolar acid in the dialysate fluid.

16. The fluid of claim 15 wherein the dilution product is formed by combining an aqueous diluent with the alkalizer buffer concentrate and the acid concentrate.

17. The fluid of claim 15 wherein about 1.6 to 3.3 parts of the alkalizer buffer and about 30-40 parts of aqueous diluent are combined with each part of acid concentrate.

18. A dialysis fluid for use in an approximately 45X hemodialysis machine, resistant to the formation of a precipitate during dialysis, which comprises:
    (a) about 90-155 mEq/L sodium ion; and
    (b) 25-45 mEq/L bicarbonate ion; which is formed by combining:
        (i) an alkalizer/buffer containing a source of alkalinity consisting essentially of aqueous sodium bicarbonate; and
        (ii) an acid concentrate comprising an acid selected from the group consisting of hydrochloric acid, acetic acid, citric acid, lactic acid, pyruvic acid, formic acid, fumaric acid, succinic acid, and mixtures thereof.

19. The fluid of claim 18 wherein the dilution product is formed by combining the acid concentrate with the aqueous diluent and then with the alkalizer/buffer.

20. The fluid of claim 19 wherein about 1.6 to 3.3 part of the alkalizer/buffer and about 35-45 parts of the aqueous diluent are combined with each part of the acid concentrate.

21. The fluid of claim 18 wherein the acid concentrate further comprises magnesium ion and calcium ion in an amount sufficient to result in the addition of about 0.1 mEq/L, independently, of each ion in the dialysate fluid.

22. A dialysis fluid for use in an approximately 45X machine, resistant to the formation of a bicarbonate precipitate during dialysis, which comprises an aqueous dilution product comprising about 90-155 mEq/L sodium ion, 25-45 mEq/L bicarbonate ion, an a physiologically acceptable concentrate of a solute selected from the group consisting of calcium ion, magnesium ion and dextrose, which is formed by combining:
        (i) an alkalizer/buffer containing a source of alkalinity consisting essentially of about 50-90 grams of sodium bicarbonate per liter of alkalizer/buffer; and
        (ii) an acid concentrate comprising hydrochloric acid in sufficient amount to result in an addition of at least 3 millimolar hydrochloric acid in the dialysate fluid.

23. The fluid of claim 22 wherein the dilution product is formed by combining the acid concentrate with the aqueous diluent and then with the alkalizer/buffer.

24. The fluid of claim 23 wherein about 1.6 to 3.3 parts of the alkalizer/buffer and about 35-50 parts of aqueous diluent are combined with each part of acid concentrate.

25. The fluid of claim 22 wherein the acid concentrate further comprises 0.1 mEq/L magnesium ion and 0.1 mEq/L calcium ion.

26. A method of hemodialysis of human blood which comprises introducing human blood into the interior of a hollow fiber hemodialysis membrane in an approximately 45X hemodialysis machine and contacting the exterior of the membrane with a dialysis fluid, resistant to the formation of a precipitate during dialysis which comprises an aqueous dilution product formed by combining:
        (i) an alkalizer/buffer containing a source of alkalinity consisting essentially of aqueous bicarbonate; and
        (ii) an acid concentrate comprising an acid in sufficient amount to result in an addition of at least 3 millimolar acid in the dialysate fluid withdrawing the dialysate from the membrane and returning the

11

blood to a patient.

27. The method of claim 26 wherein the dilution product is formed by combining an aqueous diluent with an acid concentrate and the alkalizer/buffer.

28. The method of claim 26 wherein the dialysate fluid is contacted with a membrane at a rate of about 200 to 1000 milliliters of fluid per minute.

29. The method of claim 26 wherein the acid concentrate further comprises magnesium ion and calcium ion in an amount sufficient to result in the addition of about 0.1 to 6 mEq/L, independently, of each ion in the dialysate fluid.

30. A two package concentrate for a 45X dialysis machine that can be diluted with purified water to form a physiologically compatible dialysate fluid, which comprises:
    (a) a first package comprising an aqueous alkalizer buffer comprising a bicarbonate salt; and
    (b) an acid concentrate comprising a source of acid in sufficient concentration to provide at least 3 millimoles acid in the final diluted dialysate fluid.

31. The concentrate of claim 30 wherein the bicarbonate salt is present at a concentration of about 40 to 95 grams per liter.

32. A two package concentrate for a 36.83X dialysis machine that can be diluted with purified water to form a physiologically compatible dialysate fluid, which comprises:
    (a) a first package comprising an aqueous alkalizer buffer comprising a bicarbonate salt; and
    (b) an acid concentrate comprising a source of acid in sufficient concentration to provide at least 5 millimoles acid in the final diluted dialysate fluid.

33. The concentrate of claim 32 wherein the bicarbonate salt is present at a concentration of about 40 to 95 grams per liter.